# EUROPEAN PATENT APPLICATION

(11) **EP 4 793 579 A1**
(43) Date of publication of application: **19.08.2026**
(21) Application number: 25846468.4
(22) Date of filing: 14.04.2025
(51) Int. Cl.: F25D 23/12

(54) **REFRIGERATOR STERILIZATION METHOD AND APPARATUS, REFRIGERATOR, DEVICE, MEDIUM, AND PROGRAM PRODUCT**

(30) Priority: 31.07.2024 CN 202411049233
(71) Applicant: Hefei Midea Refrigerator Co., Ltd., Hefei, Anhui 230088 (CN); Hefei Hualing Co., Ltd., Hefei, Anhui 230601 (CN); Midea Group Co., Ltd., Foshan, Guangdong 528311 (CN)
(72) Inventor: LIU, Xuezi, Hefei, Anhui 230601 (CN); WANG, Zhe, Hefei, Anhui 230601 (CN); ZHANG, Xiaofeng, Hefei, Anhui 230601 (CN)
(74) Representative: Ran, Handong
(86) International application number: PCT/CN2025/088870
(87) International publication number: WO 2026/026020

(57) **Abstract**

The present application relates to the technical field of smart home appliances, and provides a refrigerator sterilization method and apparatus, a refrigerator, a device, a medium, and a program product. The method comprises: upon determining that the standby duration of a pulse function module is greater than a first preset duration, generating a sterilization signal; and sending the sterilization signal to the pulse function module, wherein the sterilization signal is used for instructing the pulse function module to emit pulsed light in a refrigerator according to a sterilization mode. In the present application, the standby duration of the pulse function module is measured, and when the refrigerator has not been sterilized for a duration exceeding the preset duration, the sterilization signal for triggering the pulse function module to perform sterilization is automatically generated. In this way, when users forget to conduct sterilization or find manually starting sterilization too troublesome, the pulse function module is controlled to perform automatic sterilization in the refrigerator, thereby preventing bacterial growth on food during refrigeration and effectively guaranteeing cleanliness of both the food and the interior of the refrigerator.

## Description

### CROSS-REFERENCES TO RELATED APPLICATIONS

The present application claims priority to Chinese application No. 202411049233.0 filed on July 31, 2024, entitled "Refrigerator Sterilization Method and Apparatus, Refrigerator, Device, Medium, and Program Product", which is hereby incorporated by reference in its entirety.

### FIELD

The present application relates to the field of smart home appliances, and more particularly to a sterilization method and apparatus for a refrigerator, a refrigerator, a device, a medium, and a program product.

### BACKGROUND

With the continuous development of refrigerator technology, a sterilization function has become an important function of a refrigerator.

When a traditional refrigerator provides the sterilization function, a user is required to manually trigger a start button of a sterilization module or manually turn on the sterilization function through an application installed on a user terminal each time sterilization is performed.

However, in practical applications, many users often forget to turn on the sterilization function or feel that manually turning on the sterilization function each time is too troublesome, resulting in the inability to sterilize foods inside the refrigerator, causing bacterial growth and seriously affecting the cleanliness of the foods and an interior of the refrigerator.

### BRIEF SUMMARY

The present application provides a sterilization method for a refrigerator. By timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

The present application further provides a sterilization apparatus for a refrigerator, a refrigerator, a device, a medium, and a program product.

A sterilization method for a refrigerator according to an embodiment of the present application, performed by a controller, includes:
determining that a standby duration of a pulse function module is greater than a first preset duration, generating a sterilization signal; and
sending the sterilization signal to the pulse function module, where the sterilization signal is used for indicating the pulse function module to emit pulse light inside the refrigerator according to a sterilization mode.

In the sterilization method for the refrigerator according to the embodiment of the present application, by timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

According to an embodiment of the present application, sending the sterilization signal to the pulse function module includes:
determining that a door of the refrigerator is in a closed state, and sending the sterilization signal to the pulse function module; or
determining that a door of the refrigerator is in an open state, and sending the sterilization signal to the pulse function module after waiting for the door of the refrigerator to be closed.

According to an embodiment of the present application, determining that the door of the refrigerator is in the open state, and sending the sterilization signal to the pulse function module after waiting for the door of the refrigerator to be closed includes:
determining that the door of the refrigerator is in the open state, and generating a light-up signal; and
sending the light-up signal to an indicator light, where the light-up signal is used for indicating the indicator light to stay constantly on.

According to an embodiment of the present application, after sending the sterilization signal to the pulse function module, the method further includes:
determining that a door of the refrigerator is in an open state, and sending a sterilization pause signal to the pulse function module, where the sterilization pause signal is used for indicating the pulse function module to pause emitting the pulse light; and
after the door of the refrigerator is in a closed state, sending a sterilization resume signal to the pulse function module, where the sterilization resume signal is used for indicating the pulse function module to continue emitting the pulse light according to the sterilization mode.

According to an embodiment of the present application, the sterilization mode includes:
after receiving the sterilization signal or the sterilization resume signal, entering, by the pulse function module, a working preparation state; and
after a duration during which the pulse function module enters the working preparation state exceeds a second preset duration, emitting, by the pulse function module, the pulse light inside the refrigerator.

According to an embodiment of the present application, after sending the sterilization signal to the pulse function module, the method further includes:
determining that the pulse function module has completed sterilization and resetting the standby duration to zero; or
determining that a user triggers a sterilization end operation and resetting the standby duration to zero.

According to an embodiment of the present application, after determining that the user triggers the sterilization end operation and resetting the standby duration to zero, the method further includes:
determining an illumination ratio based on a ratio of a number of illumination that the pulse function module emits the pulse light to a preset number of illumination for the sterilization mode;
determining a third preset duration based on a product of the first preset duration and the illumination ratio; and
updating the first preset duration with the third preset duration.

A sterilization method for a refrigerator according to an embodiment of the present application, performed by a pulse function module, includes:
receiving a sterilization signal, where the sterilization signal is generated and sent from a controller when a standby duration of the pulse function module exceeds a first preset duration; and
emitting, in response to the sterilization signal, pulse light inside the refrigerator according to a sterilization mode.

A sterilization apparatus for a refrigerator according to an embodiment of the present application, deployed in a controller, includes:
a signal generating module, configured for determining that a standby duration of a pulse function module is greater than a first preset duration, generating a sterilization signal; and
a signal sending module, configured for sending the sterilization signal to the pulse function module, where the sterilization signal is used for indicating the pulse function module to emit pulse light inside the refrigerator according to a sterilization mode.

A sterilization apparatus for a refrigerator according to an embodiment of the present application, deployed in a pulse function module, includes:
a signal receiving module, configured for receiving a sterilization signal, where the sterilization signal is generated and sent from a controller when a standby duration of the pulse function module exceeds a first preset duration; and
a signal response module, configured for emitting, in response to the sterilization signal, pulse light inside the refrigerator according to a sterilization mode.

A refrigerator according to an embodiment of the present application includes:
a controller configured for performing any of the sterilization methods for the refrigerator described above, and
a pulse function module configured for performing any of the sterilization methods for the refrigerator described above; and
any of the sterilization apparatuses for the refrigerator described above.

An electronic device according to an embodiment of the present application, includes a memory, a processor, and a computer program stored on the memory and executable on the processor, where the processor, when executing the computer program, performs any of the sterilization methods for the refrigerator described above.

A non-transitory computer-readable storage medium according to an embodiment of the present application stores a computer program, where any of the sterilization methods for the refrigerator described above is performed when the computer program is executed by a processor.

A computer product according to an embodiment of the present application includes a computer program, where the computer program is executed by a processor to perform any of the sterilization methods for the refrigerator described above.

One or more of the above solutions in the embodiments of the present application have at least one of the following effects: by timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

Additional aspects and advantages of the present application is set forth in part in the description which follows and, in part, is apparent from the description, or may be learned by practice of the present application.

### BRIEF DESCRIPTION OF THE DRAWINGS

To more clearly illustrate solutions in the embodiments of the present application or the related art, the drawings that need to be used in the descriptions of the embodiments or the related art will be briefly described below. The drawings in the following description are only certain embodiments of the present application, and for those skilled in the related art, other drawings may be obtained based on these drawings without any creative work.
FIG. 1 is a first schematic flowchart of a sterilization method for a refrigerator according to the present application;
FIG. 2 is a second schematic flowchart of a sterilization method for a refrigerator according to the present application;
FIG. 3 is a third schematic flowchart of a sterilization method for a refrigerator according to the present application;
FIG. 4 is a first schematic structural diagram of a sterilization apparatus for a refrigerator according to the present application;
FIG. 5 is a second schematic structural diagram of a sterilization apparatus for a refrigerator according to the present application;
FIG. 6 is a schematic structural diagram of a refrigerator according to the present application; and
FIG. 7 is a schematic structural diagram of an electronic device according to the present application.

### DETAILED DESCRIPTION

Implementations of the present application are further described in detail below with reference to the drawings and embodiments. The following embodiments are intended to illustrate the present application, but are not intended to limit the scope of the present application.

In the description of the present specification, description with reference to the terms "some embodiment," "in an embodiment," "an example," "specific example," "some examples" and the like, refers to that specific features, structures, materials or characteristics described in combination with an embodiment or an example are included in at least one embodiment or example of the embodiments of the present application. In this specification, schematic expressions of the above terms do not necessarily refer to the same embodiment or example. Also, the described specific features, structures, materials or characteristics can be combined in any one or more embodiments or examples in a suitable manner. In addition, those skilled in the art may combine the different embodiments or examples described in this specification, as well as the features of the different embodiments or examples, without conflicting each other.

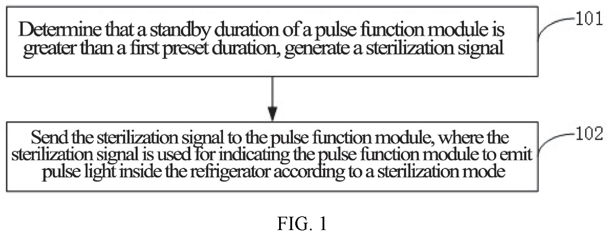

Sterilization methods and apparatuses for a refrigerator, a refrigerator, a device, a medium, and a program product are described below with reference to Fig. 1 to Fig. 7.

FIG. 1 is a first schematic flowchart of a sterilization method for a refrigerator according to the present application. As shown in FIG. 1, the sterilization method for the refrigerator may be performed by a refrigerator and includes but not limited to the following step 101 and step 102.

Step 101, determining that a standby duration of a pulse function module is greater than a first preset duration, generating a sterilization signal.

The pulse function module is a function module connected to the refrigerator and sterilizes foods the refrigerator inside by emitting pulse light.

The standby duration refers to a cumulative duration during which the pulse function module is in a non-sterilization standby state.

The first preset duration is a predetermined interval between two times at which the refrigerator is sterilized.

When the controller detects that the standby duration of the pulse function module exceeds the first preset duration, it means that the refrigerator has not been sterilized for a period of time, and a user does not manually turn on a sterilization function within the first preset duration. At this time, the controller automatically generates a sterilization signal to trigger the pulse function module to enter a sterilization state.

In an embodiment, the standby duration is timed from when the refrigerator is powered on, or from a time at which the pulse function module completes a previous sterilization process.

In an embodiment, upon completing the previous sterilization process, the pulse function module sends a sterilization end signal to the refrigerator, and the refrigerator starts timing from when receiving the sterilization end signal.

In an embodiment, an illumination sensor may be provided inside the refrigerator to sense the pulse light emitted from the pulse function module based on a working principle of the pulse function module which sterilizes foods by emitting pulse light. The controller then starts timing when the illumination sensor senses the last pulse light in the previous sterilization process.

In an embodiment, the controller starts timing after a time required for sterilization has elapsed from a start time of the previous sterilization based on the sterilization time required by the sterilization mode and the start time of the previous sterilization.

In some embodiments, the first preset duration may be determined based on at least one of the following factors: a capacity and a structure of the refrigerator, the type and/or actual quantity of foods stored in the refrigerator, a sterilization frequency, storage requirements of the foods, a real-time temperature inside the refrigerator, an actual operating state of the refrigerator, etc.

For example, the first preset duration may be set to 8 hours, 12 hours, or 16 hours, etc.

In an embodiment, during refrigerator operation, the first preset duration may be shortened or lengthened based on factors such as the type and/or actual quantity of foods stored in the refrigerator, and a completion condition of the previous sterilization.

For example, a factory default setting for the first preset duration is 12 hours. If there is too may foods stored inside the refrigerator, the first preset duration may be lengthened to 16 hours. In an embodiment, if the door of the refrigerator is not opened or no new food is added after the previous sterilization process, the 12 hours can be shortened to 8 hours.

In an embodiment, the pulse function module includes a xenon lamp.

In an embodiment, the pulse function module is either detachably or non-detachably connected to the refrigerator.

It should be noted that the pulse function module may be mounted anywhere in the refrigerator where food sterilization is required. For example, the pulse function module may be mounted on a refrigerator compartment, or in a drawer within the refrigerator compartment; or may be mounted on the top wall or side wall of the refrigerator compartment. The specific mounting position of the pulse function module may be determined based on actual sterilization needs, and does not be limited herein.

Step 102, sending the sterilization signal to the pulse function module, where the sterilization signal is used for indicating the pulse function module to emit pulse light inside the refrigerator according to a sterilization mode.

The sterilization mode includes, but not limited to, an operation mode included of at least one of the following parameters: a number of time that the pulse function module emits the pulse light inside the refrigerator, an emitting frequency, an illumination intensity, or a duration of a single illumination.

For example, the sterilization mode is an operation mode that the pulse function module emits the pulse light inside the refrigerator continuously for 180 seconds at a frequency of once per second to sterilize the foods.

For another example, the sterilization mode is an operation mode that the pulse function module emits the pulse light inside the refrigerator continuously for 90 seconds at a frequency of twice per second to sterilize the foods.

After generating the sterilization signal, the controller sends the sterilization signal to the pulse function module. Upon receiving the signal, the pulse function module emits the pulse light inside the refrigerator according to a preset sterilization mode to sterilize the foods inside the refrigerator.

For refrigerators equipped with pulse function modules implementing sterilization, the user is often required to manually turn on the sterilization function. Furthermore, after completing one sterilization process, the user must manually turn on the function again for a subsequent sterilization process. In other words, the user must manually turn on the sterilization function for each sterilization process. In this situation, many users forget to turn on the sterilization function or feel that manually turning on the sterilization function each time is too troublesome, resulting in the inability to sterilize foods inside the refrigerator, causing bacterial growth and seriously affecting the cleanliness of the foods and an interior of the refrigerator.

In the sterilization method for the refrigerator provided by the present application, by timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

Based on the above embodiment, sending the sterilization signal to the pulse function module includes:
determining that a door of the refrigerator is in a closed state, and sending the sterilization signal to the pulse function module; or
determining that a door of the refrigerator is in an open state, and sending the sterilization signal to the pulse function module after waiting for the door of the refrigerator to be closed.

When the standby duration of the pulse function module exceeds the first preset duration, the controller generates the sterilization signal. However, before sending the sterilization signal to the pulse function module, the controller first obtains a determination result of whether the door of the refrigerator is in a closed state, and determine a timing of sending the sterilization signal to the pulse function module based on the determination result.

Upon determining that the door of the refrigerator is in the closed state, the controller may directly send the sterilization signal to pulse function module.

Upon determining that the door of the refrigerator is in an open state, the controller pauses sending the sterilization signal to the pulse function module, and after determining the door of the refrigerator is in the closed state, sends the sterilization signal to the pulse function module to trigger the pulse function module to emit the pulse light inside the refrigerator according to the sterilization mode.

In an embodiment, when determining the door of the refrigerator is in the open state, the controller determines the door of the refrigerator is in the closed state after receiving a door-closing signal generated from a sudden change in the illumination intensity of the ambient sensed from the illumination sensor located inside the refrigerator.

When the door of the refrigerator is in the open state, ambient light may enter an interior of the refrigerator, and an illuminating lamp inside the refrigerator is turned on. When changing from the open state to the closed state, the door of the refrigerator blocks the ambient light, and the illuminating lamp inside the refrigerator is turned off and a signal generated from a sudden change in the illumination intensity of the ambient sensed from the illumination sensor may then serve as the door-closing signal generated when the door of the refrigerator changes from the open state to the closed state.

In an embodiment, the door-closing signal may be a signal generated when a door frame of the refrigerator changes from a pressure-released state to a pressed state detected by a pressure sensor located on the door frame of the refrigerator.

In the sterilization method for the refrigerator provided in the present application, the controller determines whether the door of the refrigerator is closed only after the user does not manually turn on the sterilization function for a certain period of time and triggers the pulse function module to perform sterilization upon determining the door of the refrigerator is in the closed state, avoiding the pulse light emitted from the pulse function module to illuminate the user who is using the refrigerator with the open door to ensure sterilization safety.

Based on the above embodiment, determining that the door of the refrigerator is in the open state, and sending the sterilization signal to the pulse function module after waiting for the door of the refrigerator to be closed includes:
determining that the door of the refrigerator is in the open state, and generating a light-up signal; and
sending the light-up signal to an indicator light, where the light-up signal is used for indicating the indicator light to stay constantly on.

Before the sterilization function needs to be turned on automatically, upon determining that the door of the refrigerator is not closed, the controller generates the light-up signal and sends the light-up signal to the indicator light. Upon receiving the light-up signal, the indicator light stays constantly on to prompt the user that the sterilization function is about to automatically turned on and that the user is required to use the refrigerator and close the door as soon as possible.

In an embodiment, the light-up signal generated from the controller may also be sent to the pulse function module. Upon receiving the light-up signal, the pulse function module forwards the light-up signal to the indicator light connected to the pulse function module.

Furthermore, after the user closes the door of the refrigerator, the controller sends a door-closing signal to the pulse function module. Upon receiving the door-closing signal, the pulse function module, in addition to emitting pulse light inside the refrigerator, can also control the indicator light to change from being on to being off.

In an embodiment, the indicator light is integrated into the pulse function module. Upon receiving a light-up signal, the pulse function module controls the indicator light to stay constantly on.

In an embodiment, the indicator light is electrically connected to the pulse function module, rather than integrated into the pulse function module. In this case, mounting positions of the pulse function module and the indicator light may differ.

For example, if the pulse function module is mounted on the top wall inside the refrigerator, the emitted pulse light will more easily illuminate all stored foods. Simultaneously, the indicator light may be mounted on the inner wall of the refrigerator facing the door, more easily attracting the user's attention and better prompting them that the sterilization function is about to automatically be turned on.

In another embodiment, the pulse function module is connected with a voice prompt playback device.

Before the sterilization function needs to be turned on automatically, upon determining that the door of the refrigerator is not closed, the controller generates a playback signal and sends the playback signal to the pulse function module. Upon receiving the playback signal, the voice prompt playback device connected to the pulse function module begins to operate, plays a pre-stored voice message indicating that the sterilization function is about to automatically be turned on, prompting the user that the user is required to use the refrigerator and close the door as soon as possible.

In the sterilization method for the refrigerator provided in the present application, when the refrigerator is about to automatically turn on its sterilization function and the user is still using the refrigerator, the indicator light is controlled to stay constantly on for prompting the user to use the refrigerator and close the door as soon as possible, ensuring safe sterilization of food after the user closes the door of the refrigerator.

Based on the above embodiment, after sending the sterilization signal to the pulse function module, the method further includes:
determining that a door of the refrigerator is in an open state, and sending a sterilization pause signal to the pulse function module, where the sterilization pause signal is used for indicating the pulse function module to pause emitting the pulse light; and
after the door of the refrigerator is in a closed state, sending a sterilization resume signal to the pulse function module, the sterilization resume signal is used for indicating the pulse function module to continue emitting the pulse light according to the sterilization mode.

After starting the automatic sterilization function, it generally takes tens of seconds to several minutes for the refrigerator to complete the sterilization of foods. If the user happens to open the door of the refrigerator while the refrigerator is using pulse light for sterilization but has not yet completed sterilization, the sterilization using the pulse light needs to be automatically paused.

When a user opens the door of the refrigerator, the controller generates the sterilization pause signal based on a door-opening signal generated when the door changes from the closed state to the open state detected by sensors such as a pressure sensor, an illumination sensor, and an infrared sensor and sends the door-opening signal to the pulse function module. Upon receiving the sterilization pause signal, the pulse function module pauses emitting the pulse light.

Furthermore, when the user closes the door again during pausing the sterilization, the controller receives the door-losing signal generated when the door changes from the open state to the closed state. The controller generates a sterilization resume signal and sends the sterilization resume signal to the pulse function module. Upon receiving the sterilization resume signal, the pulse function module resumes emitting the pulse light according to the sterilization mode.

For example, if the sterilization mode is an operation mode that the pulse function module continuously operates for 180 seconds at a frequency of once per second, and the pulse function module has already operated for 80 seconds when the user opens the door of the refrigerator, that is, emits the pulse light for 80 times. Therefore, after the user closes the door again, the pulse function module will continue operating for another 100 seconds, emits the pulse light for 100 times.

The refrigerator is provided with an indicator light and/or a voice playback device. If the door of the refrigerator is suddenly opened by a user while the pulse function module is emitting the pulse light to sterilize the foods, the indicator light and/or the voice playback device stays constantly on and play a voice prompt indicating that automatic sterilization is in progress to prompt the user that the refrigerator is automatically sterilizing and the user is required to close the door of the refrigerator as soon as possible.

In the sterilization method for the refrigerator provided in the present application, sterilization is automatically paused if the refrigerator is automatically sterilizing and the user opens the door. Sterilization is resumed after the user closes the door, preventing the pulse light emitted from the pulse function module from illuminating the user who needs to open the door and causing adverse effects, and ensuring sterilization safety.

Based on the above embodiment, the sterilization mode includes:
after receiving the sterilization signal or the sterilization resume signal, entering, by the pulse function module, a working preparation state; and
after a duration during which the pulse function module enters the working preparation state exceeds a second preset duration, emitting, by the pulse function module, the pulse light inside the refrigerator.

Upon receiving or resuming the sterilization signal, the pulse function module does not immediately emit the pulse light. Instead, the pulse function module enters a preparation state and only emits the pulse light to sterilize the foods inside the refrigerator after a second preset duration has elapsed in this preparation state.

The second preset duration may be determined based on one or more factors of performance parameters of the pulse function module, the capacity of the refrigerator, and the types and quantities of foods stored in the refrigerator and other factors.

For example, the second preset duration may be set to 3 seconds, 4 seconds, 8 seconds, 10 seconds, etc.

In the sterilization method for the refrigerator provided in the present application, compared to a method where the pulse function module immediately emits the pulse light for sterilization upon receiving or resuming the sterilization signal, the sterilization mode is set to enable the pulse function module to enter a preparation state for a certain period of time after the sterilization signal is received or resumed before the sterilization is started, which may avoid the user being exposed to the pulse light when suddenly opening the door of the refrigerator and allow the pulse function module to accumulate energy in a sufficient charging time, improving the sterilization effect.

Based on the above embodiment, after sending the sterilization signal to the pulse function module, the method further includes:
determining that the pulse function module has completed sterilization and resetting the standby duration to zero; or
determining that a user triggers a sterilization end operation and resetting the standby duration to zero.

Generally, sterilizing the refrigerator may be performed in the following two ways.

In one way, after the user manually turns on sterilization, i.e., the user manually triggers a power button on the pulse function module, or selects an operation to start sterilization in an application installed on a user terminal, the pulse function module then receives a sterilization signal generated by itself, or receives a sterilization signal generated from the application installed on the user terminal in response to selecting, by the user, an operation to start sterilization and forwarded by the refrigerator, and emits the pulse light based on the sterilization signal for sterilization.

In the other way, the refrigerator automatically turns on sterilization, i.e., upon the standby duration of the pulse function module exceeds a first preset duration, the controller generates and sends a sterilization signal to the pulse function module to trigger the pulse function module to emit pulse light inside the refrigerator according to the sterilization mode.

Regardless of whether the user manually turns on sterilization or the refrigerator automatically turns on sterilization, after the pulse function module completes sterilization, or even before the pulse function module completes sterilization but the user manually stops the ongoing sterilization process of the refrigerator via a button or application operation (i.e., triggering a sterilization end operation), the controller receives a sterilization end signal, resets the standby duration data of the pulse function module to zero, and retimes the standby duration of the pulse function module and restarts the automatic sterilization process if the standby duration of the pulse function module exceeds a first preset duration.

In an embodiment, the sterilization end signal is generated and sent to the controller by the pulse function module after the sterilization is completed.

In an embodiment, the sterilization end signal is generated by the controller based on a time required for sterilization and a start time of a current sterilization operation, after the time required for sterilization has elapsed from the start time of the current sterilization operation.

In an embodiment, the sterilization end signal is generated and sent to the refrigerator by the pulse function module when the user triggers a turn-on/turn-off button of the pulse function module (i.e., triggering the sterilization end operation). The turn-on/turn-off button can be either a wired control button integrated into the pulse function module or a virtual button on a control panel of the refrigerator.

In an embodiment, the sterilization end signal is generated by the user terminal and sent to the controller after the user selects to end the sterilization operation in the application of the user terminal.

In the sterilization method for the refrigerator provided in the present application, the standby duration of the pulse function module is recalculated after each sterilization process is completed and ended and after each sterilization process, even if the user forgets the next sterilization process or does not turn on the sterilization function since the user feel manually turning on sterilization to be too troublesome, the refrigerator can automatically perform the subsequent food sterilization process, preventing bacterial growth on foods during refrigeration and effectively ensuring the cleanliness of foods and the interior of the refrigerator.

Based on the above embodiment, after determining that the user triggers the sterilization end operation and resetting the standby duration to zero, the method further includes:
determining an illumination ratio based on a ratio of a number of illumination that the pulse function module emits the pulse light to a preset number of illumination for the sterilization mode;
determining a third preset duration based on a product of the first preset duration and the illumination ratio; and
updating the first preset duration with the third preset duration.

When the controller determines that the user triggers a sterilization end operation and the pulse function module stops emitting the pulse light, the controller determines, based on the sterilization end signal, a number of illumination that the pulse function module emits the pulse light according to the sterilization mode, obtains an illumination ratio of the number of times of illumination to the preset number of illumination for the sterilization mode and obtain the third preset duration by multiplying the irradiation ratio by the first preset duration. Then, the first preset duration is updated with the calculated third preset duration, and the standby duration is reset to zero. The standby duration of the pulse function module is retimed until the standby duration of the pulse function module reaches the third preset duration. At this point, the controller generates a new sterilization signal and sends the new sterilization signal to the pulse function module to trigger the pulse function module to emit the pulse light inside the refrigerator according to the sterilization mode, for sterilizing the foods inside the refrigerator.

In an embodiment, if the sterilization end signal is generated by the pulse function module and sent to the controller after the user triggers the turn-on/turn-off button of the pulse function module, the sterilization end signal contains information about the number of times of illumination that the pulse function module emits the pulse light during the current sterilization process. Upon receiving the sterilization end signal, the controller parses the number of times of illumination from the signal and determines the illumination ratio based on the ratio between the number of times of illumination and the preset number of illumination.

In an embodiment, if the sterilization end signal is generated by the user terminal and sent to the controller after the user selects to terminate sterilization in the application on the user terminal, the controller receives the sterilization end signal and forwards the sterilization end signal to the pulse function module, controls the pulse function module to stop sterilization and obtains information about the number of times of illumination fed back by the pulse function module. In another embodiment, the controller receives the sterilization end signal and forwards the sterilization end signal to the pulse function module, controls the pulse function module to stop sterilization while simultaneously determining information about the number of times of illumination that the pulse function module emits the pulse light during the current sterilization process based on the illumination signal received from the illumination sensor, and further determines the illumination ratio based on the ratio between the number of times of illumination and the preset number of illumination.

In an embodiment, the third preset duration is expressed by the following formula: ${T}_{3} = {T}_{1} \times \frac{{S}_{1}}{S}$ where *T*₃ is the third preset duration, *T*₁ is the first preset duration, *S*₁ is the number of times of illumination that the pulse function module emits the pulse light according to the sterilization mode, and *S* is the preset number of illumination for the sterilization mode.

For example, assuming the first preset duration *T*₁ is 12 hours and the preset number of illumination for the sterilization mode *S* is 180 times. When the pulse function module has performed illumination for 72 times during the current sterilization process, the user ends the sterilization process via the application of the user terminal and the number of times of illumination *S*₁ is 72 times and the third preset duration *T*₃ is calculated to be 4.8h based on 12h×(72÷180). Therefore, after receiving the sterilization end signal, if the standby duration of the pulse function module exceeds 4.8h, the controller generates a new sterilization signal and sends the new sterilization signal to the pulse function module to trigger a new round of pulse light emission sterilization according to the preset number of illumination being 180 of the sterilization mode.

In the sterilization method for the refrigerator provided in the present application, in case that the user manually ends the current sterilization process, resulting in incomplete sterilization, the controller recalculates the standby duration condition for generating the sterilization signal based on the number of times of illumination that the pulse light is emitted and the preset number of illumination to shorten an interval between the incomplete sterilization process and the start of the next sterilization process, preventing incomplete sterilization and bacterial growth on foods during refrigeration if the user does not actively turn on sterilization within a certain period of time, effectively ensuring the cleanliness of food and the interior of the refrigerator.

The following embodiment is provided to better illustrate the complete process of the sterilization method for the refrigerator of the present application.

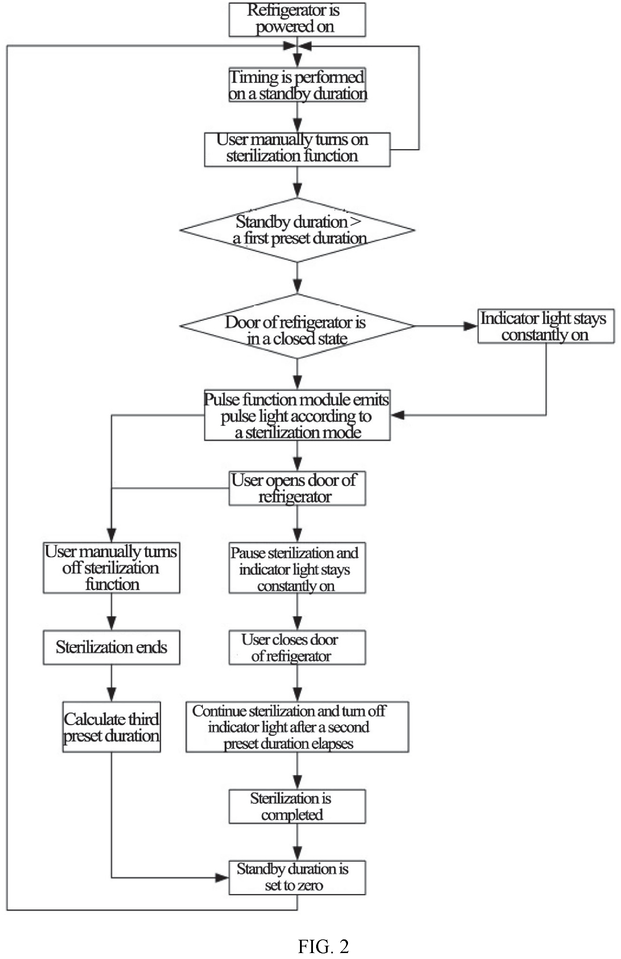

FIG. 2 is a second schematic flowchart of a sterilization method for a refrigerator according to the present application. As shown in Fig. 2, after the refrigerator is powered on, timing is performed on the standby duration of the pulse function module. During the first preset duration from the start of timing, if the user manually turns on the sterilization function through the turn-on button of the pulse function module or the application of the user terminal, the standby duration is retimed. If the standby duration of the pulse function module exceeds the first preset duration, a sterilization signal is generated.

Before sending the sterilization signal to the pulse function module, the controller determines whether the door of the refrigerator is in a closed state. If the door of the refrigerator is in the closed state, the controller sends the sterilization signal to the pulse function module, controls the pulse function module to emit the pulse light according to the sterilization mode. If the door of the refrigerator is not in a closed state, a light-up signal is generated and sent to the pulse function module, the indicator light connected to the pulse function module is triggered to stay constantly on. This lighting prompts the user that the sterilization function is about to automatically turned on, the user is required to use the refrigerator and close the door as soon as possible. The sterilization signal is only sent to the pulse function module after the door-closing signal is received, and the indicator light is controlled to be then turned off from a constant-on state.

If the user opens the door of the refrigerator during the sterilization process of the pulse function module, the controller generates a sterilization signal and a light-up signal and sends both the sterilization signal and the light-up signal to the pulse function module. The controller controls the pulse function module to pause emitting the pulse light and keep the indicator light to stay constantly on, prompting the user that the refrigerator is automatically sterilizing and the door should be closed as soon as possible.

After the user closes the door of the refrigerator, the controller generates a sterilization resume signal and sends the sterilization resume signal to the pulse function module. Upon receiving the sterilization resume signal, the pulse function module enters a preparation state. After remaining in the preparation state for a second preset duration, the pulse function module resume emitting the pulse light inside the refrigerator and turns off the indicator light. After sterilization is completed, the standby duration is reset to zero and the standby duration is retimed, allowing for manual sterilization if the user does not turn on the sterilization function again within a first preset duration.

If the user manually turns off the sterilization function during the sterilization process of the pulse function module, the current sterilization process has ended before completing the sterilization. The controller determines a third preset duration based on the number of times of illumination that the pulse function module emits the pulse light during the current sterilization process and a preset number of illumination preset for the sterilization mode and determines the third preset duration as a new first preset duration. The controller resets the standby duration to zero, and retimes the standby duration to perform manual sterilization when the user does not turn on the sterilization function again within a duration exceeding the third preset duration.

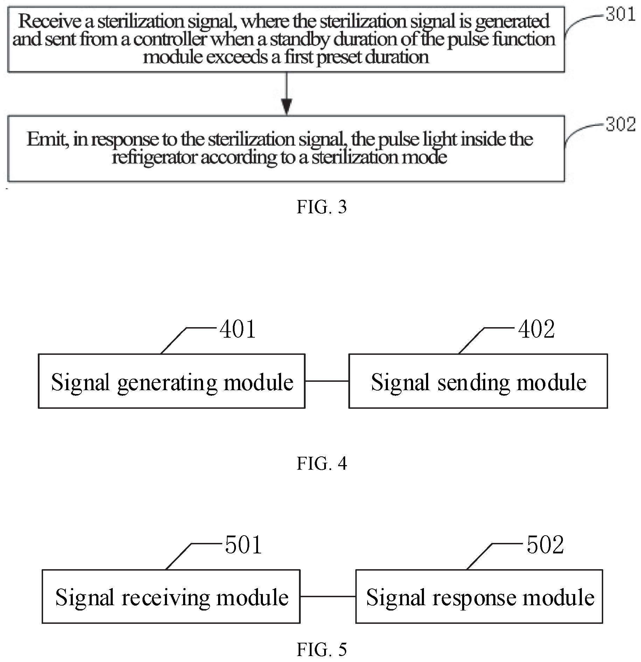

FIG. 3 is a third schematic flowchart of third schematic sterilization method for a refrigerator according to the present application. As shown in FIG. 3, the sterilization method for the refrigerator may be performed by a pulse function module and includes but not limited to the following step 301 and step 302:
step 301, receiving a sterilization signal, where the sterilization signal is generated and sent from a controller when a standby duration of the pulse function module exceeds a first preset duration; and
step 302, emitting, in response to the sterilization signal, pulse light inside the refrigerator according to a sterilization mode.

It should be noted that the sterilization method for the refrigerator performed by the pulse function module provided in the present application corresponds one-to-one with the sterilization method for the refrigerator performed by the controller described in any of the above embodiments during specific operation; therefore, the embodiment is not described in detail.

In the sterilization method for the refrigerator provided by the present application, by timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

FIG. 4 is a first schematic structural diagram of a sterilization apparatus for a refrigerator according to the present application. As shown in FIG. 4, the sterilization apparatus for the refrigerator may be deployed in a controller and includes but not limited to a signal generating module 401 and a signal sending module 402.

The signal generating module 401 is configured for determining that a standby duration of a pulse function module is greater than a first preset duration, generating a sterilization signal.

The signal sending module 402 is configured for sending the sterilization signal to the pulse function module, where the sterilization signal is used for indicating the pulse function module to emit pulse light inside the refrigerator according to a sterilization mode.

It should be noted that the sterilization apparatus for the refrigerator provided in the present application may perform the sterilization method for the refrigerator performed by the refrigerator described in any of the above embodiments and the embodiment is not described in detail.

In the sterilization apparatus for the refrigerator provided by the present application, by timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

FIG. 5 is a second schematic structural diagram of a sterilization apparatus for a refrigerator according to the present application. As shown in FIG. 5, the sterilization apparatus for the refrigerator may be deployed in a pulse function module and includes but not limited to a signal receiving module 501 and a signal response module 502.

The signal receiving module 501 is configured for receiving a sterilization signal, where the sterilization signal is generated and sent from a controller included in a refrigerator when a standby duration of the pulse function module exceeds a first preset duration.

The signal response module 502 is configured for emitting, in response to the sterilization signal, pulse light inside the refrigerator according to a sterilization mode.

It should be noted that the sterilization apparatus for the refrigerator provided in the present application may perform the sterilization method for the refrigerator performed by the pulse function module described in any of the above embodiments and the embodiment is not described in detail.

In the sterilization apparatus for the refrigerator provided by the present application, by timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

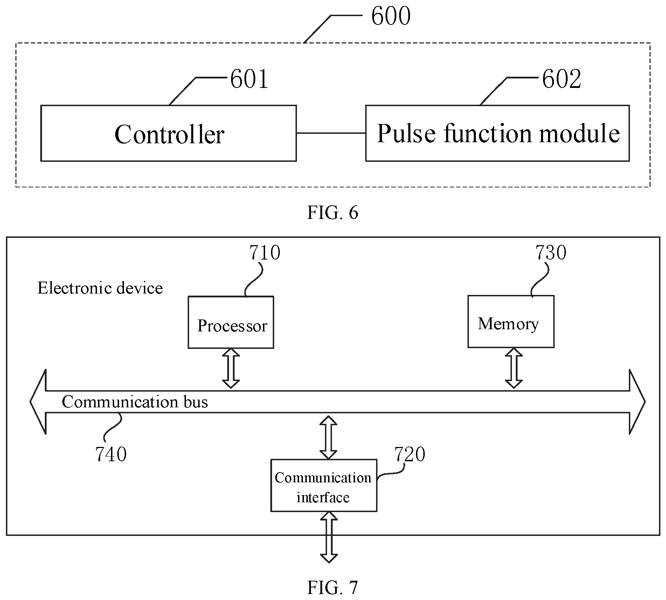

FIG. 6 is schematic structural diagram of a refrigerator according to the present application. As shown in FIG. 6, the refrigerator 600 includes a controller 601 and a pulse function module 602.

The controller 601 performs the sterilization method for the refrigerator performed by the controller provided in any of the above embodiments. The sterilization method for the refrigerator includes, but not limited to, the following steps: determining that a standby duration of a pulse function module 602 is greater than a first preset duration, generating a sterilization signal; and sending the sterilization signal to the pulse function module 602, where the sterilization signal is used for indicating the pulse function module 602 to emit pulse light inside the refrigerator according to a sterilization mode.

The pulse function module 602 performs the sterilization method for the refrigerator performed by the pulse function module provided in any of the above embodiments. The sterilization method for the refrigerator includes, but not limited to, the following steps: receiving a sterilization signal, where the sterilization signal is generated and sent from a controller 601 when a standby duration of the pulse function module 602 exceeds a first preset duration; and emitting, in response to the sterilization signal, pulse light inside the refrigerator according to a sterilization mode.

In the refrigerator provided by the present application, by timing a standby duration of a pulse function module, a sterilization signal for triggering the pulse function module to perform sterilization is automatically generated when the sterilization is not performed inside the refrigerator for a preset duration and the pulse function module is controlled to automatically sterilize the refrigerator when users forget to sterilize or feel that manually turning on the sterilization is too troublesome, preventing bacterial growth on food during refrigeration and effectively ensuring the cleanliness of foods and an interior of the refrigerator.

FIG. 7 is a schematic structural diagram of an electronic device according to the present application. As shown in FIG. 7, the electronic device may include a processor 710, a communication interface 720, a memory 730, and a communication bus 740. The processor 710, the communication interface 720, and the memory 730 communicate with each other through the communication bus 740. The processor 710 can invoke a logic instruction in the memory 730 to perform the sterilization method for the refrigerator performed by the controller provided in any of the above embodiments. The sterilization method for the refrigerator includes, but not limited to, the following steps: determining that a standby duration of a pulse function module is greater than a first preset duration, generating a sterilization signal; and sending the sterilization signal to the pulse function module, where the sterilization signal is used for indicating the pulse function module to emit pulse light inside the refrigerator according to a sterilization mode; or
perform sterilization method for the refrigerator performed by the pulse function module provided in any of the above embodiments. The sterilization method for the refrigerator includes, but not limited to, the following steps: receiving a sterilization signal, where the sterilization signal is generated and sent from a controller when a standby duration of the pulse function module exceeds a first preset duration; and emitting, in response to the sterilization signal, pulse light inside the refrigerator according to a sterilization mode.

In addition, the logic instruction in the memory 730 described above may be implemented in the form of a software functional unit and may be stored in a computer readable storage medium while being sold or used as a separate product. Based on such understanding, the solution of the present disclosure or a part of the technical solution, which is essential or contributes to the related art, may be embodied in the form of a software product, which is stored in a storage medium, including several instructions to cause a computer device (which may be a personal computer, server, or network device, etc.) to perform all or part of the steps of the methods described in various embodiments of the present application. The storage medium described above includes: U disk, a mobile hard disk, a read-only memory (ROM), a random access memory (RAM), a magnetic disk, or an optical disk, and the like.

The present application discloses a computer program product, including a computer program stored on a non-transitory computer-readable storage medium. The computer program includes a program instruction, and when executed by a computer, the computer can perform the sterilization method for the refrigerator performed by the controller provided in any of the above embodiments. The sterilization method for the refrigerator includes, but not limited to, the following steps: determining that a standby duration of a pulse function module is greater than a first preset duration, generating a sterilization signal; and sending the sterilization signal to the pulse function module, where the sterilization signal is used for indicating the pulse function module to emit pulse light inside the refrigerator according to a sterilization mode; or
perform sterilization method for the refrigerator performed by the pulse function module provided in any of the above embodiments. The sterilization method for the refrigerator includes, but not limited to, the following steps: receiving a sterilization signal, where the sterilization signal is generated and sent from a controller when a standby duration of the pulse function module exceeds a first preset duration; and emitting, in response to the sterilization signal, pulse light inside the refrigerator according to a sterilization mode.

An embodiment of the present application further provides a non-transitory computer-readable storage medium storing a computer program. The computer program is executed by a processor to perform the sterilization method for the refrigerator performed by the controller provided in any of the above embodiments. The sterilization method for the refrigerator includes, but not limited to, the following steps: determining that a standby duration of a pulse function module is greater than a first preset duration, generating a sterilization signal; and sending the sterilization signal to the pulse function module, where the sterilization signal is used for indicating the pulse function module to emit pulse light inside the refrigerator according to a sterilization mode; or
perform sterilization method for the refrigerator performed by the pulse function module provided in any of the above embodiments. The sterilization method for the refrigerator includes, but not limited to, the following steps: receiving a sterilization signal, where the sterilization signal is generated and sent from a controller when a standby duration of the pulse function module exceeds a first preset duration; and emitting, in response to the sterilization signal, pulse light inside the refrigerator according to a sterilization mode.

The apparatuses embodiments described above are merely illustrative, where the units described as separate components may or may not be physically separate, and the components displayed as units may or may not be physical units, that is, may be located at the same place or be distributed to multiple network units. Some or all of the modules may be selected according to actual needs to achieve the purpose of the solution of the present embodiment. Those skilled in the art can understand and implement the embodiments described above without paying creative labors.

Through the description of the embodiments above, it can be clearly understood for those skilled in the art that the various embodiments can be implemented by means of software and a necessary general hardware platform, and of course, by hardware. Based on such understanding, the solution of the present application or a part of the solution, which is essential or contributes to the related art, may be embodied in the form of a software product, which is stored in a storage medium such as ROM/RAM, magnetic Discs, optical discs, etc., including several instructions to cause a computer device (which may be a personal computer, server, or network device, etc.) to perform various embodiments or a part of the methods described in various embodiments.

Finally, it should be noted that the above embodiments are only used to illustrate the present application, but not to limit the present application. Although the application has been described in detail with reference to the embodiments, those skilled in the art should understand that various combinations, modifications, or equivalent replacements of the technical solutions of the application do not depart from the scope of the solutions of the application, and should all cover the scope of the solutions of the various embodiments of the present application.

## Claims

1. A sterilization method for a refrigerator, performed by a controller, comprising:
determining that a standby duration of a pulse function module is greater than a first preset duration, and generating a sterilization signal; and
sending the sterilization signal to the pulse function module, wherein the sterilization signal is used for instructing the pulse function module to emit pulse light inside the refrigerator based on a sterilization mode.

2. The method of claim 1, wherein said sending the sterilization signal to the pulse function module comprises:
determining that a door of the refrigerator is in a closed state, and sending the sterilization signal to the pulse function module; or
determining that a door of the refrigerator is in an open state, and sending the sterilization signal to the pulse function module after waiting until the door of the refrigerator is closed.

3. The method of claim 2, wherein said determining that the door of the refrigerator is in the open state, and sending the sterilization signal to the pulse function module after waiting until the door of the refrigerator is closed comprises:
determining that the door of the refrigerator is in the open state, and generating a light-up signal; and
sending the light-up signal to an indicator light, wherein the light-up signal is used for instructing the indicator light to stay constantly on.

4. The method of claim 1, wherein after said sending the sterilization signal to the pulse function module, the method further comprises:
determining that a door of the refrigerator is in an open state, and sending a sterilization pause signal to the pulse function module, wherein the sterilization pause signal is used for instructing the pulse function module to pause emitting the pulse light; and
after the door of the refrigerator enters a closed state, sending a sterilization resume signal to the pulse function module, wherein the sterilization resume signal is used for instructing the pulse function module to continue emitting the pulse light based on the sterilization mode.

5. The method of claim 4, wherein the sterilization mode comprises:
after receiving the sterilization signal or the sterilization resume signal, entering, by the pulse function module, a working preparation state; and
after a duration for which the pulse function module remains in the working preparation state exceeds a second preset duration, emitting, by the pulse function module, the pulse light inside the refrigerator.

6. The method of claim 1, wherein after said sending the sterilization signal to the pulse function module, the method further comprises:
determining that the pulse function module has completed sterilization and resetting the standby duration to zero; or
determining that a user triggers a sterilization end operation and resetting the standby duration to zero.

7. The method of claim 6, wherein after said determining that the user triggers the sterilization end operation and resetting the standby duration to zero, the method further comprises:
determining an illumination ratio based on a ratio of the number of times that the pulse function module emits the pulse light to a preset number of illuminations for the sterilization mode;
determining a third preset duration based on a product of the first preset duration and the illumination ratio; and
updating the first preset duration with the third preset duration.

8. A sterilization method for a refrigerator, performed by a pulse function module, comprising:
receiving a sterilization signal, wherein the sterilization signal is generated and sent from a controller when a standby duration of the pulse function module exceeds a first preset duration; and
emitting, in response to the sterilization signal, pulse light inside the refrigerator based on a sterilization mode.

9. A sterilization apparatus for a refrigerator, deployed in a controller, comprising:
a signal generating module, configured to determine that a standby duration of a pulse function module is greater than a first preset duration, and generate a sterilization signal; and
a signal sending module, configured to send the sterilization signal to the pulse function module, wherein the sterilization signal is used for instructing the pulse function module to emit pulse light inside the refrigerator based on a sterilization mode.

10. A sterilization apparatus for a refrigerator, deployed in a pulse function module, comprising:
a signal receiving module, configured to receive a sterilization signal, wherein the sterilization signal is generated and sent from a controller when a standby duration of the pulse function module exceeds a first preset duration; and
a signal response module, configured to emit, in response to the sterilization signal, pulse light inside the refrigerator based on a sterilization mode.

11. A refrigerator, comprising:
a controller configured to perform the sterilization method for the refrigerator of any one of claims 1 to 7;
a pulse function module configured to perform the sterilization method for the refrigerator of claim 8; and
the sterilization apparatus for the refrigerator of claim 9 and the sterilization apparatus for the refrigerator of claim 10.

12. An electronic device, comprising a memory, a processor, and a computer program stored on the memory and executable on the processor, wherein the processor, when executing the computer program, performs the sterilization method for the refrigerator of any one of claims 1 to 7 or the sterilization method for the refrigerator of claim 8.

13. A non-transitory computer-readable storage medium having a computer program stored thereon, wherein the sterilization method for the refrigerator of any one of claims 1 to 7 or the sterilization method for the refrigerator of claim 8 is performed when the computer program is executed by a processor.

14. A computer program product, comprising a computer program, wherein the computer program, when executed by a processor, performs the sterilization method for the refrigerator of any one of claims 1 to 7 or the sterilization method for the refrigerator of claim 8.
